# EUROPÄISCHE PATENTSCHRIFT

(11) **EP 2 280 644 B1**
(45) Veröffentlichungstag und Bekanntmachung des Hinweises auf die Patenterteilung: **23.11.2011**
(21) Anmeldenummer: 09757267.1
(22) Anmeldetag: 02.06.2009
(51) Int. Cl.: A61B 5/05, A61B 6/00, G01N 22/00

(54) **MESSVORRICHTUNG UND VERFAHREN ZUR MIKROWELLENBASIERTEN UNTERSUCHUNG**
MEASURING INSTRUMENT AND METHOD FOR MICROWAVE-BASED EXAMINATIONS
DISPOSITIF DE MESURE ET PROCEDE D'EXAMEN PAR MICRO-ONDES

(30) Priorität: 02.06.2008 DE 102008026438; 16.12.2008 DE 102008062484
(43) Veröffentlichungstag der Anmeldung: 09.02.2011
(73) Patentinhaber: Rohde & Schwarz GmbH & Co. KG, 81671 München (DE)
(72) Erfinder: LEIBFRITZ, Martin, 81825 München (DE)
(74) Vertreter: Körfer, Thomas
(86) Internationale Anmeldenummer: PCT/EP2009/003925
(87) Internationale Veröffentlichungsnummer: WO 2009/146884

(56) Entgegenhaltungen:
- EP-A- 0 804 900
- EP-A- 1 834 667
- WO-A-2005/053531
- DE-A1- 3 020 015
- US-A- 5 334 141
- US-A1- 2006 241 410

## Beschreibung

Die Erfindung betrifft eine Messvorrichtung und ein Verfahren zur mikrowellenbasierten Untersuchung.

Herkömmlich werden zur mikrowellenbasierten Untersuchung von Objekten Antennen eingesetzt, welche durch einen großen Luftbereich von dem zu untersuchenden Objekt entfernt sind. Dies dient dem Erreichen von Fernfeldbedingungen. So kann eine Beeinflussung der Antennen durch das individuelle Untersuchungsobjekt vermieden werden. Nachteilhaft ist jedoch, dass jeder Materialübergang Reflexionen hervorruft, welche die Untersuchung stören.

Alternativ werden Antennen eingesetzt, welche direkt auf dem zu untersuchenden Objekt aufliegen. Es tritt dann jedoch eine Verkopplung zwischen dem Untersuchungsobjekt und der Antenne auf. So zeigt die US 5,704,355 ein System zur Detektion von Brustkrebs, welches eine Antenne im direkten Kontakt mit der Brust einsetzt. Nachteilhaft ist hier zusätzlich eine geringe Positionierungsgenauigkeit der Antenne. So kann die Brust während der Untersuchung gegenüber der Antenne verrutschen.

Der Erfindung liegt die Aufgabe zu Grunde, eine Messvorrichtung und ein Verfahren zur mikrowellenbasierten Untersuchung von Objekten zu schaffen, welche eine hohe Qualität der Untersuchung erbringen und einen geringen Aufwand erfordern.

Die Aufgabe wird erfindungsgemäß für die Vorrichtung durch die Merkmale des unabhängigen Anspruchs 1 und für das Verfahren durch die Merkmale des unabhängigen Anspruchs 6 gelöst. Vorteilhafte Weiterbildungen sind Gegenstand der hierauf rückbezogenen Unteransprüche.

Eine erfindungsgemäße Messvorrichtung verfügt über einen Mikrowellensender, einen Mikrowellenempfänger, zumindest eine Antenne, eine Steuerungseinrichtung und eine dielektrische Verlängerung. Die dielektrische Verlängerung ist zwischen der Antenne und einem zu untersuchenden Objekt angeordnet. Die Steuerungseinrichtung steuert den Mikrowellensender und den Mikrowellenempfänger. Der Mikrowellensender sendet mittels der Antenne und der dielektrischen Verlängerung ein Mikrowellensignal in das zu untersuchende Objekt. Das zu untersuchende Objekt streut das Mikrowellensignal. Der Mikrowellenempfänger empfängt mittels der Antenne und der dielektrischen Verlängerung das gestreute Mikrowellensignal. Die Länge und Dielektrizitätskonstante der dielektrischen Verlängerung sind dabei derart dimensioniert, dass das zu untersuchende Objekt im Fernfeld der Antenne liegt. So kann eine genaue Untersuchung bei kleinen Abmessungen der Messvorrichtung erreicht werden.

Die dielektrische Verlängerung verfügt vorteilhafterweise über eine Dielektrizitätskonstante von ε > 2, bevorzugt von ε > 3, besonders bevorzugt von ε > 4. Die Verlängerung verfügt vorteilhafterweise über eine Länge von 20cm bis 120cm, bevorzugt von 30cm bis 100cm, besonders bevorzugt von 40cm bis 80cm. So können besonders kleine Abmessungen der Messvorrichtung erreicht werden.

Vorteilhafterweise verfügt die dielektrische Verlängerung über einen seitlich angeordneten Mikrowellenabsorber. Störungen durch seitliche Streuungen der Mikrowellensignale werden so vermieden.

Bevorzugt besteht der Mikrowellenabsorber aus einem stark dämpfenden, nicht reflektierenden Material. So werden Störungen durch seitliche Streuungen der Mikrowellensignale bei kleinen Abmessungen der Messvorrichtung vermieden.

Das zu untersuchende Objekt ist bevorzugt eine Brust einer Patientin. Die Brust ist bevorzugt zwischen der Patientin und der Verlängerung angeordnet. Zwischen der Brust und der Verlängerung befindet sich bevorzugt im Wesentlichen keine Luft. So wird eine flache, in ihrer Dicke weitgehend homogene Brust erreicht. Auch ein reflexionsarmer Übergang der Strahlung wird so erreicht. Eine hohe Genauigkeit der Messung wird so erreicht, insbesondere zur Detektion von Brustkrebs.

Die Messvorrichtung beinhaltet bevorzugt zumindest eine zweite Antennenanordnung. Die erste Antennenanordnung und die zweite Antennenanordnung sind bevorzugt einzelne Antennen oder Antennenarrays. So kann eine weitere Erhöhung der Genauigkeit erreicht werden.

Der Mikrowellensender sendet bevorzugt mittels der ersten Antennenanordnung ein Mikrowellensignal in das zu untersuchende Objekt. Der Mikrowellenempfänger empfängt bevorzugt mittels der zweiten Antennenanordnung das gestreute Signal. Alternativ sendet der Mikrowellensender mittels der zweiten Antennenanordnung ein Mikrowellensignal in das zu untersuchende Objekt. Der Mikrowellenempfänger empfängt dann bevorzugt mittels der ersten Antennenanordnung das gestreute Signal. So kann auf aufwendige Signalverarbeitung zur Trennung von einer einzelnen Antenne gesendeter und von der gleichen Antenne empfangenen Signalen verzichtet werden.

Die zweite Antennenanordnung nimmt bevorzugt gegenüber dem zu untersuchenden Objekt nacheinander mehrere Positionen ein. So kann eine hohe Ortsauflösung erreicht werden.

Nachfolgend wird die Erfindung anhand der Zeichnung, in der vorteilhafte Ausführungsbeispiele der Erfindung dargestellt sind, beispielhaft beschrieben. In der Zeichnung zeigen:
- Fig. 1: ein erstes Ausführungsbeispiel der erfindungsgemäßen Messvorrichtung;
- Fig. 2: eine Detailansicht des ersten Ausführungsbeispiels der erfindungsgemäßen Messvorrichtung;
- Fig. 3: ein zweites Ausführungsbeispiel der Messvorrichtung;
- Fig. 4: eine erste Detailansicht des zweiten Ausführungsbeispiels der Messvorrichtung;
- Fig. 5: eine zweite Detailansicht des zweiten Ausführungsbeispiels der Messvorrichtung;
- Fig. 6: ein drittes Ausführungsbeispiel der Messvorrichtung;
- Fig. 7: ein viertes Ausführungsbeispiel der Messvorrichtung;
- Fig. 8: ein fünftes Ausführungsbeispiel der Messvorrichtung;
- Fig. 9: ein Flussdiagramm eines ersten Ausführungsbeispiels des erfindungsgemäßen Verfahrens, und
- Fig. 10: ein Flussdiagramm eines zweiten Ausführungsbeispiels des Verfahrens.

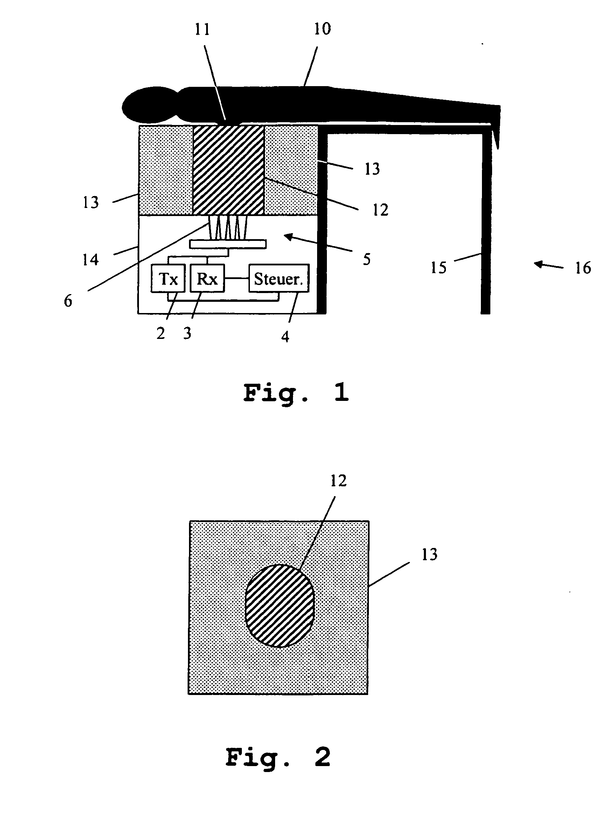

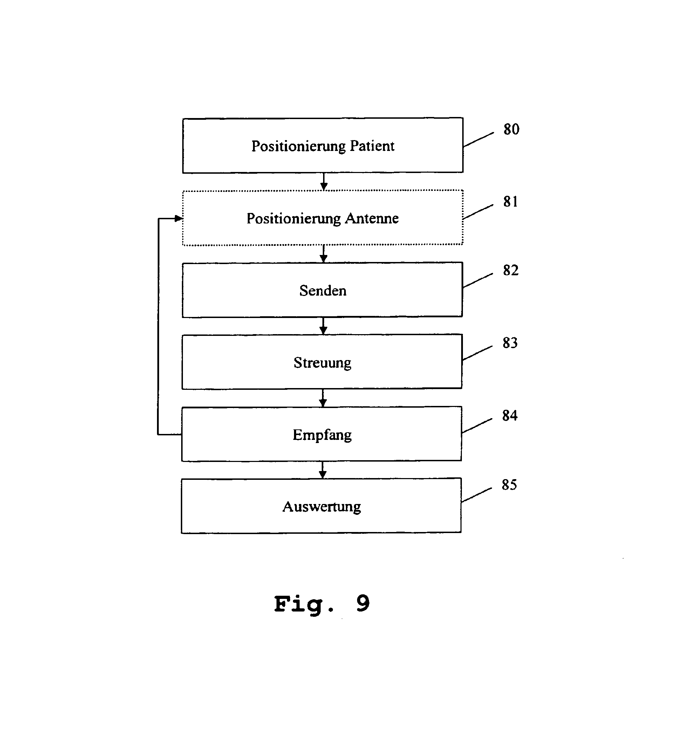

Zunächst wird anhand der Fig. 1 - 2 der Aufbau und die Funktionsweise der erfindungsgemäßen Vorrichtung nach Anspruch 1 gezeigt. Abschließend wird mittels Fig. 9 - 10 die Funktionsweise verschiedener Ausführungsbeispiele des Verfahrens dargestellt. Identische Elemente wurden in ähnlichen Abbildungen zum Teil nicht wiederholt dargestellt und beschrieben.

Fig. 1 zeigt ein erstes Ausführungsbeispiel der erfindungsgemäßen Messvorrichtung. Eine Patientin 10 liegt auf einer Patientenauflage 16. Die Patientenauflage 16 beinhaltet dabei neben einem Tisch 15 ein Gehäuse 14, eine dielektrische Verlängerung 12 und einen Absorber 13. Das Gehäuse 14 ist unterhalb der dielektrischen Verlängerung 12 und des Absorber 13 angeordnet. Der Tisch 15 ist neben dem Gehäuse 14, der dielektrischen Verlängerung 12 und dem Absorber 13 angeordnet. Die dielektrische Verlängerung 12 und der Absorber 13 sind dabei in einer Schnittdarstellung gezeigt.

Die Brust 11 der Patientin 10 befindet sich zwischen ihrem übrigen Körper und der dielektrischen Verlängerung 12 oberhalb eines ersten Endes der dielektrischen Verlängerung. Die Brust 11 wird dabei durch das Körpergewicht der Patientin 10 komprimiert. Dies führt zu einer weitgehend flachen, gleichmäßig dicken Form der Brust 11. So kann eine Korrekturrechnung, welche Reflexionen an der Oberfläche der Brust eliminiert, vereinfacht durchgeführt werden. Auch eine aufrecht sitzende Position der Patientin 10 oder eine Rückenlage bei aufgesetzter Verlängerung 12 sind möglich.

Das Gehäuse 14 beinhaltet einen Mikrowellensender 2 einen Mikrowellenempfänger 3 eine Steuerungseinrichtung 4 und ein Antennenarray 5. Das Antennenarray 5 besteht dabei aus einer Mehrzahl von einzelnen Antennen 6.

Die Brust 11 der Patientin 10 ist in diesem Ausführungsbeispiel das zu untersuchende Objekt. Die Steuerungseinrichtung 4 steuert den Mikrowellensender 2 derart, das dieser mittels des Antennenarrays 5 ein Mikrowellensignal in das zu untersuchende Objekt sendet. Das Mikrowellensignal wird dabei von dem Antennenarray 5 direkt in die Verlängerung 12 gesendet. Die Verlängerung 12 weist dabei eine hohe Dielektrizitätskonstante und eine geringe Dämpfung auf. So wird die effektive Wellenlänge des Mikrowellensignals innerhalb der dielektrischen Verlängerung 12 verringert. Das Mikrowellensignal durchläuft so eine höhere Anzahl an Wellenlängen während es sich durch die Verlängerung bewegt, als dies bei einem Durchgang durch die Luft geschieht. So wird eine Entkopplung des Antennenarrays 5 von dem zu untersuchenden Objekt auch bei geringer Distanz erreicht. D.h. die Antenneneigenschaften werden nicht von den Eigenschaften des zu untersuchenden Objekts beeinflusst. Eine solche Entkopplung tritt auf, wenn Fernfeldbedingungen herrschen. Dies ist der Fall, wenn das Mikrowellensignal zwischen der Antenne 6 und der Brust 11 zumindest sechs Wellenlängen zurückgelegt hat.

Der Einsatz der Verlängerung 12 ermöglicht die Nutzung hochfrequenter Mikrowellenstrahlung, ohne eine große Distanz zwischen Antenne und zu untersuchendem Objekt einhalten zu müssen.

Um Störungen durch von den Rändern der Verlängerung 12 reflektierten Mikrowellensignalen zu vermeiden ist die Verlängerung 12 von einem Absorber 13 umgeben. Der Absorber 13 besteht dabei aus einem mikrowellenabsorbierenden Material.

Durch die direkte Auflage der Brust 11 auf der dielektrischen Verlängerung 12 werden zusätzliche Störungen durch Restriktionen des Mikrowellensignals an dem Übergang zur Luft vermieden.

Das Mikrowellensignal wird von dem zu untersuchenden Objekt, hier der Brust 11 gestreut. Ein Teil des gestreuten Mikrowellensignals wird durch die Verlängerung 12 zurück zu dem Antennenarray 5 gestreut. Der Mikrowellenempfänger 3 wird von der Steuerungseinrichtung 4 derart gesteuert, dass dieser das zurückgestreute Mikrowellensignal empfängt.

Um eine möglichst hohe Ortsauflösung innerhalb des zu untersuchenden Objekts zu erzielen, werden unterschiedliche Sendepositionen und Empfangspositionen eingesetzt. Dies wird in diesem Ausführungsbeispiel durch Nutzung unterschiedlicher Einzelantennen 6 des Antennenarrays 5 erreicht. Abschließend bestimmt die Steuerungseinrichtung 4 aus den empfangenen Mikrowellensignalen eine Mikrowellentomographie des zu untersuchenden Objekts.

In Fig. 2 wird eine Detailansicht des ersten Ausführungsbeispiels der erfindungsgemäßen Messvorrichtung dargestellt. Dargestellt ist eine Schnittdarstellung der dielektrischen Verlängerung 12 und des Absorbers 13. Der Schnitt erfolgt dabei in horizontaler Ebene. Deutlich zu erkennen ist die an das zu untersuchende Objekt, hier die Brust 11 der Patientin 10, angepasste Form der Verlängerung 12. Der Absorber 13 umschließt die Verlängerung 12 in horizontaler Ebene vollständig. Der Absorber 13 weist eine Dicke auf, welche im Wesentlichen dem kleinsten Radius der Verlängerung 12 entspricht. So wird eine sichere Absorption der aus der Verlängerung 12 austretenden Mikrowellenstrahlung erzielt. Störungen durch Reflexionen werden so reduziert.

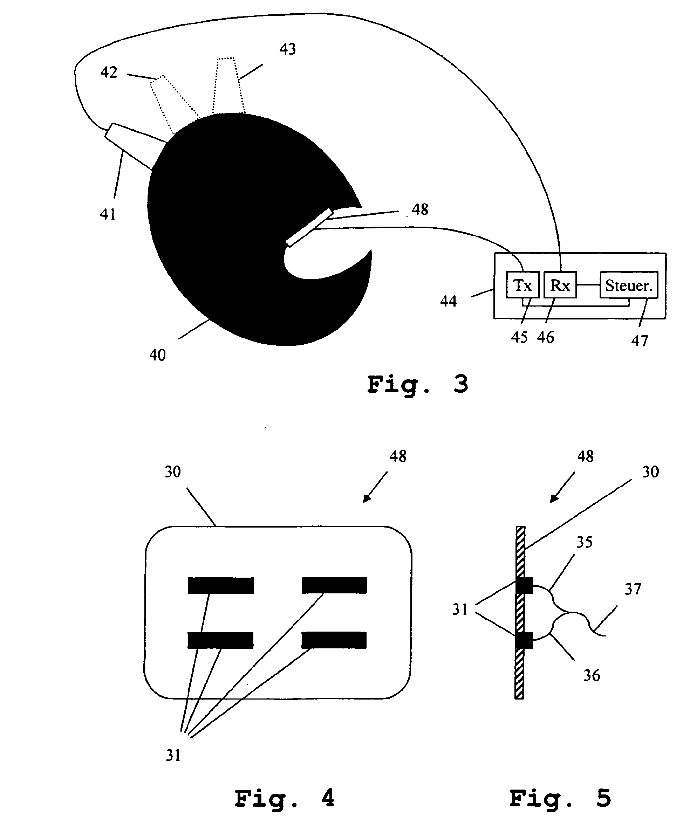

Fig. 3 zeigt ein zweites Ausführungsbeispiel der Messvorrichtung. Ein Gehäuse 44 enthält einen Mikrowellensender 45, einen Mikrowellenempfänger 46 und eine Steuerungseinrichtung 47. Die Steuerungseinrichtung 47 steuert sowohl den Mikrowellensender 45 als auch die Mikrowellenempfänger 46. Der Mikrowellensender 45 ist mit einer Antennenanordnung 48 verbunden. Die Antennenanordnung 48 befindet sich im Mund eines Patienten. Sie ist z.B. mittels eines Klebers am Gaumen im Kopf 40 des Patienten fixiert. Die Antennenanordnung 48 ist dabei zumindest teilweise flexibel und kann so an die Oberfläche des Gaumens angepasst werden. Der Mikrowellenempfänger 46 ist mit einer Antenne 41 verbunden. Die Antenne 41 steht dabei in direktem Kontakt zu der Außenseite des Kopfes 40 des Patienten. Das zu untersuchende Objekt ist hier der Kopf 40 des Patienten.

Um eine Untersuchung durchzuführen, steuert die Steuerungseinrichtung 47 den Mikrowellensender 45 derart an, dass dieser mittels der Antennenanordnung 48 ein Mikrowellensignal in den Kopf 40 des Patienten sendet. Das Mikrowellensignal wird von dem zu untersuchenden Objekt gestreut. Das gestreute Mikrowellensignal wird von dem Mikrowellenempfänger 46 mittels der Antenne 41 empfangen und an die Steuerungseinrichtung 47 weitergeleitet. Nacheinander nimmt die Antenne 41 mehrere unterschiedliche Positionen 42, 43 ein. So wird eine Ortsauflösung bei der Untersuchung erzielt. Die Steuerungseinrichtung 47 bestimmt aus den gestreuten Signalen eine zumindest zweidimensionale Mikrowellentomographie des zu untersuchenden Objekts. Alternativ zu einer Umpositionierung der Antenne 41 kann ein Antennenarray eingesetzt werden.

Besonders vorteilhaft ist bei diesem Ausführungsbeispiel, dass lediglich sehr geringe Reflexionen auftreten. Dies wird erreicht durch das Vermeiden von Luftspalten zwischen der Antennenanordnung 48 und dem Kopf 40 des Patienten und zwischen dem Kopf 40 des Patienten und der Antenne 41. Nachteilhaft an diesem Ausführungsbeispiel ist jedoch, dass auf Grund der geringen Distanz eine Verkoppelung der Antennenanordnung 48 mit dem Kopf 40 des Patienten und der Antenne 41 mit dem Kopf 40 des Patienten besteht. Eine aufwändige Kalibrierung ist somit für jedes unterschiedliche zu untersuchende Objekt, also für jeden unterschiedlichen Patienten, notwendig.

Alternativ zu dem Senden des Mikrowellensignals mittels der Antennenanordnung 48 und den Empfangen des Mikrowellensignals durch die Antenne 41 ist ein umgekehrtes Vorgehen ebenfalls möglich. Dabei wird durch Verschiebung der Antenne 41 an die mehreren Positionen 42, 43 die Sendepositionen verändert, und damit die Ortsauflösung erzielt.

In Fig. 4 wird eine erste Detailansicht des zweiten Ausführungsbeispiels der Messvorrichtung dargestellt. Die Unterseite, d.h. die in Fig. 3 am Gaumen befestigte Seite der Antennenanordnung 48, wird hier dargestellt. Sie beinhaltet einen flexiblen Träger 30 und vier Antennenelemente 31. Die Antennenelemente 31 sind dabei leitfähige Streifen auf der Unterseite des flexiblen Trägers 30.

Fig. 5 zeigt eine zweite Detailansicht des zweiten Ausführungsbeispiels der Messvorrichtung. Die Antennenanordnung 48 wird hier in einer seitlichen Schnittdarstellung gezeigt. Die Antennenelemente 31 sind auf der Oberseite des flexiblen Trägers 30 mit Signalleitungen 35, 36 verbunden, welche zu einer gemeinsamen Signalleitung 37 gebündelt sind.

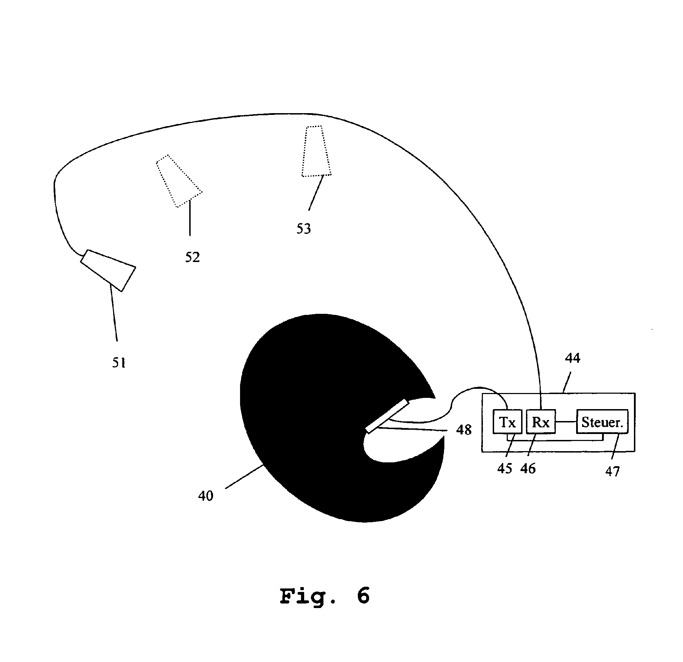

In Fig. 6 wird ein drittes Ausführungsbeispiel der Messvorrichtung dargestellt. Um die von der anhand von Fig. 3 erläuterten Verkoppelung der Antenne 41 und des Kopfes 40 des Patienten hervorgerufenen Schwierigkeiten zu beseitigen, wurde die Antenne 51, welche der Antenne 41 aus Fig. 3 entspricht, nicht direkt am Kopf 40 des Patienten positioniert. Stattdessen weist die Antenne 51 einen Abstand zu dem Kopf 40 des Patienten auf. Der Abstand ist dabei so gewählt, dass Fernfeldbedingungen herrschen.

Im Übrigen entspricht das hier dargestellte Ausführungsbeispiel dem in Fig. 3 dargestellten Ausführungsbeispiel.

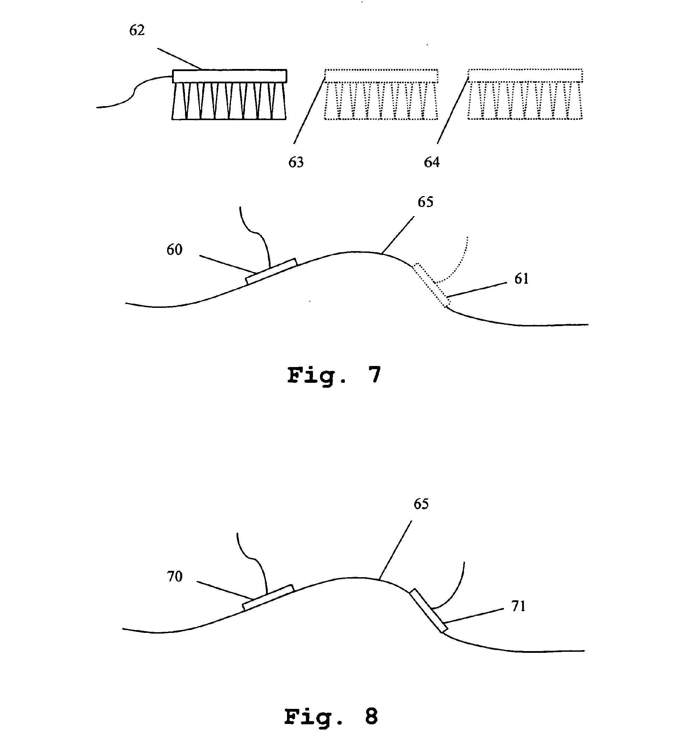

Fig. 7 zeigt ein viertes Ausführungsbeispiel der Messvorrichtung. Bei dem zu untersuchenden Objekt handelt es sich erneut um eine menschliche Brust 65. Auf der Brust 65 ist eine Antennenanordnung 60 z.B. durch Kleben angebracht. In einem Abstand zu der Brust 65, welcher Fernfeldbedingungen gewährleistet, befindet sich ein Antennenarray 62. Um eine Untersuchung vorzunehmen, wird mittels der Antennenanordnung 60 ein Mikrowellensignal in die Brust 65 gestrahlt. Das Mikrowellensignal wird von der Brust 65 gestreut. Das gestreute Mikrowellensignal wird von dem Antennenarray 62 empfangen. Aus dem empfangenen Mikrowellensignal wird eine Mikrowellentomographie der Brust 65 von einer Steuerungseinrichtung, wie in Fig. 1, Fig. 3, und Fig. 6 dargestellt, bestimmt. Um die Ortsauflösung weiter zu erhöhen, wird das Antennenarray 62 nacheinander an unterschiedliche Positionen 63, 64 gebracht. Eine weitere Erhöhung der Ortsauflösung wird erreicht, indem die Antennenanordnung 60 nach der Untersuchung an ihrer ursprünglichen Position an die zusätzliche Position 61 gebracht wird. Durch die Mehrzahl an Empfangspositionen und Sendepositionen wird eine hohe Ortsauflösung erreicht.

Durch das direkte Auffliegen der Antennenanordnung 60 auf der Brust 65 werden sehr geringe Störungen durch Reflexionen an der Haut erreicht. Eine Umkehrung der Senderrichtung ist auch bei diesem Ausführungsbeispiel möglich. So dient das Antennenarray 62 zum Senden des Mikrowellensignals, während die Antennenanordnung 60 zum Empfangen des Mikrowellensignals dient.

In Fig. 8 wird ein fünftes Ausführungsbeispiel der Messvorrichtung dargestellt. Das hier dargestellte Ausführungsbeispiel entspricht zum Teil dem in Fig. 7 dargestellten Ausführungsbeispiel. Die Antennenanordnung 70 entspricht der Antennenanordnung 60 aus Fig. 7. Anstatt eines Antennenarrays 62 aus Fig. 7 wird eine zweite Antennenanordnung 71 eingesetzt. Die zweite Antennenanordnung 71 ist dabei wie die erste Antennenanordnung 70 auf die Brust 65 aufgeklebt. Zur Durchführung einer Untersuchung wird ein Mikrowellensignal mittels der ersten Antennenanordnung 70 in die Brust 65 gesendet. Das Mikrowellensignal wird von der Brust 65 gestreut. Das gestreute Mikrowellensignal wird von der zweiten Antennenanordnung 71 empfangen. Eine Ortsauflösung wird erreicht, indem jedes Antennenelement 70, 71 über mehrere einzelne Antennen verfügt. Eine zusätzliche Erhöhung der Ortsauflösung ist durch Einsatz zusätzlicher Antennenanordnungen möglich. Auch eine Umpositionierung der lediglich zwei Antennenanordnungen 70, 71 erzielt eine Erhöhung der Ortsauflösung. Jede Umpositionierung ist jedoch mit einer Positionierungsungenauigkeit verbunden.

Auch bei diesem Ausführungsbeispiel bestimmt eine Steuerungseinrichtung, wie in Fig. 1, Fig. 3 und Fig. 6 dargestellt, eine Mikrowellentomographie aus den empfangenen Mikrowellensignalen. Auch hier ist eine Umkehrung der Senderrichtung möglich. So kann auch die Antennenanordnung 71 zum Senden des Mikrowellensignals genutzt werden, während die Antennenanordnung 70 zum Empfangen des gestreuten Mikrowellensignals genutzt wird.

Fig. 9 zeigt ein Flussdiagramm eines ersten Ausführungsbeispiels des erfindungsgemäßen Verfahrens. In einem ersten Schritt 80 wird das zu untersuchende Objekt, hier ein Patient, positioniert. Dabei wird es in direkten Kontakt mit der anhand von Fig. 1 - 2 detailliert beschriebenen dielektrischen Verlängerung gebracht. In einem optionalen zweiten Schritt 81 wird die Antenne gegenüber dem Objekt positioniert. Dieser Schritt ist bei beweglichen einzelnen Antennen relevant. Bei Nutzung eines Antennenarrays kann durch Positionierung, des Antennenarrays an unterschiedlichen Positionen die Ortsauflösung erhöht werden. In einem dritten Schritt 82 wird ein Mikrowellensignal in das zu untersuchende Objekt gesendet. In einem vierten Schritt 83 wird das Mikrowellensignal durch das zu untersuchende Objekt gestreut. In einem fünften Schritt 84 wird das gestreute Mikrowellensignal empfangen.

Der zweite Schritt 81 bis fünfte Schritt 84 werden für unterschiedliche Orte innerhalb des zu untersuchenden Objekts wiederholt. Auf den optionalen zweiten Schritt 81 kann verzichtet werden, wenn eine Ortsauflösung durch Nutzung unterschiedlicher Einzelantennen eines Antennenarrays erreicht wird. Nach Abschluss der Messungen an sämtlichen zu untersuchenden Orten innerhalb des zu untersuchenden Objekts wird in einem sechsten Schritt 85 eine Mikrowellentomographie des zu untersuchenden Objekts bestimmt.

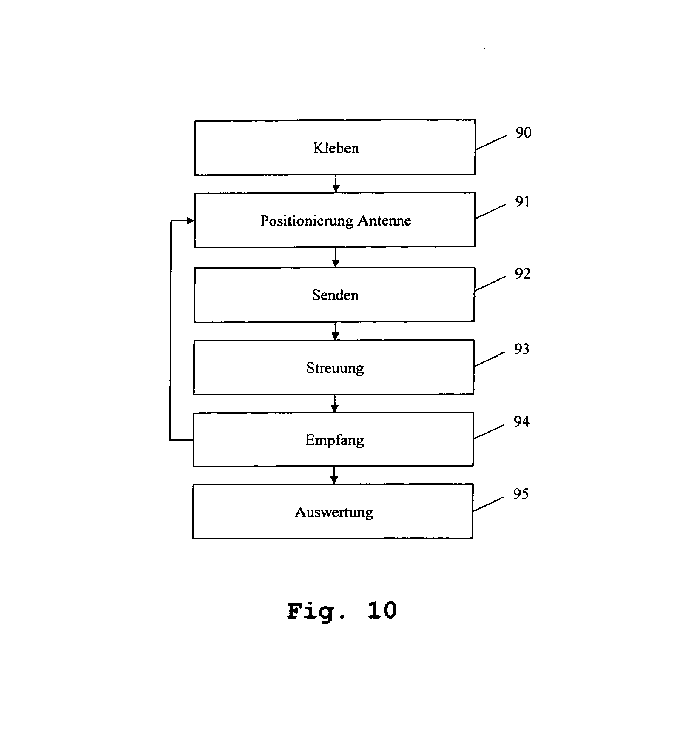

In Fig. 10 wird ein zweites Ausführungsbeispiel des Verfahrens dargestellt. In einem ersten Schritt 90 wird eine Antennenanordnung mittels Kleben an dem zu untersuchenden Objekt befestigt. In einem zweiten Schritt 91 wird das zu untersuchende Objekt, hier ein Patient, gegenüber weiteren Antennen beziehungsweise einem Antennenarray positioniert. In einem dritten Schritt 92 wird mittels der Antennenanordnung ein Mikrowellensignal in das zu untersuchende Objekt gesendet. In einem vierten Schritt 93 wird das Mikrowellensignal durch das zu untersuchende Objekt gestreut. In einem fünften Schritt 94 wird das gestreute Mikrowellensignal durch die weiteren Antennen bzw. durch das Antennenarray empfangen.

Der zweite Schritt 91 bis fünfte Schritt 94 werden für mehrere unterschiedliche Orte innerhalb des untersuchenden Objekts wiederholt. Eine Auf diese Weise wird eine verbesserte Ortsauflösung erzielt. Auf eine Bewegung der weiteren Antennen beziehungsweise des Antennenarrays gegenüber dem zu untersuchenden Objekt kann verzichtet werden, wenn die Ortsauflösung durch Nutzung mehrerer unterschiedlicher Einzelantennen eines Antennenarrays erzielt wird. In einem sechsten Schritt 95 wird mittels der empfangenen Mikrowellensignale eine Mikrowellentomographie erstellt.

Auch bei diesem Ausführungsbeispiel ist eine Umkehrung der Signalrichtung möglich. So kann das Mikrowellensignal auch durch die weiteren Antennen bzw. durch das Antennenarray gesendet und durch die aufgeklebte Antennenanordnung empfangen werden. Bei den zu untersuchenden Objekten kann es sich um beliebige Objekte, insbesondere um lebendiges oder totes menschliches oder tierisches Gewebe handeln.

Die Erfindung ist nicht auf das dargestellte Ausführungsbeispiel beschränkt. Wie bereits erwähnt, können unterschiedliche Objekte untersucht werden. Auch die Nutzung unterschiedlicher Antennenanzahlen ist möglich.

## Patentansprüche

1. Messvorrichtung mit einem Mikrowellensender (2), einem Mikrowellenempfänger (3), zumindest einer Antenne (6),
einer Steuerungseinrichtung (4) und einer dielektrischen Verlängerung (12),
wobei die dielektrische Verlängerung (12) zwischen der Antenne (6) und einem zu untersuchenden Objekt (10) angeordnet ist,
wobei die Steuerungseinrichtung (4) den Mikrowellensender (2) und den Mikrowellenempfänger (3) steuert,
wobei der Mikrowellensender (2) ein Mikrowellensignal mittels der Antenne (6) und der dielektrischen Verlängerung (12) in das zu untersuchende Objekt (11) sendet,
wobei das zu untersuchende Objekt (11) das Mikrowellensignal streut,
wobei der Mikrowellenempfänger (3) das gestreute Mikrowellensignal mittels der Antenne (6) und der dielektrischen Verlängerung (12) empfängt,
wobei die Länge und Dielektrizitätskonstante der dielektrischen Verlängerung (12) derart dimensioniert sind, dass das zu untersuchende Objekt (11) im Fernfeld der Antenne (6) liegt, und
wobei das Fernfeld der Antenne (6) nach einem Zurücklegen von sechs Wellenlängen durch die dielektrische Verlängerung (12) durch das Mikrowellensignal erreicht ist.

2. Messvorrichtung nach Anspruch 1,
**dadurch gekennzeichnet,**
**dass** die dielektrische Verlängerung (12) eine Dielektrizitätskonstante von ε > 2, bevorzugt von ε > 3, besonders bevorzugt von ε > 4 hat, und
**dass** die dielektrische Verlängerung (12) eine Länge von 20cm bis 120cm, bevorzugt von 30cm bis 100cm, besonders bevorzugt von 40cm bis 80cm hat.

3. Messvorrichtung nach Anspruch 1 oder 2,
**dadurch gekennzeichnet,**
**dass** die dielektrische Verlängerung (12) über einen seitlich angeordneten Mikrowellenabsorber (13) verfügt.

4. Messvorrichtung nach Anspruch 3,
**dadurch gekennzeichnet,**
**dass** der Mikrowellenabsorber (13) aus einem stark dämpfenden, nicht reflektierenden Material besteht.

5. Messvorrichtung nach einem der Ansprüche 1 bis 4,
**dadurch gekennzeichnet,**
**dass** das zu untersuchende Objekt eine Brust (11) einer Patientin (10) ist,
**dass** die Brust an einem Ende der dielektrischen Verlängerung (12) angeordnet ist, und
**dass** sich zwischen der Brust (11) und der dielektrischen Verlängerung (12) im Wesentlichen keine Luft befindet.

6. Verfahren zur Untersuchung eines Objekts mit einem Mikrowellensender (2), einem Mikrowellenempfänger (3), zumindest einer Antenne (6), einer Steuerungseinrichtung (4) und einer dielektrischen Verlängerung (12),
wobei der Mikrowellensender (2) und der Mikrowellenempfänger (3) von der Steuerungseinrichtung (4) gesteuert werden,
wobei die folgenden Schritte ausgeführt werden:
- Senden eines Mikrowellensignals durch den Mikrowellensender (2) mittels der Antenne (6);
- Verlängerung des effektiven Wegs des Mikrowellensignals durch die dielektrische Verlängerung (12), wobei die Länge und Dielektrizitätskonstante der dielektrischen Verlängerung (12) derart dimensioniert sind, dass das zu untersuchende Objekt (11) im Fernfeld der Antenne (6) liegt, wobei das Fernfeld der Antenne (6) nach einem Zurücklegen von sechs Wellenlängen durch die dielektrische Verlängerung (12) durch das Mikrowellensignal erreicht ist;
- Streuen des Mikrowellensignals durch das zu untersuchende Objekt (11), und
- Empfangen des gestreuten Mikrowellensignals durch den Mikrowellenempfänger (3) mittels der Antenne (6).

7. Verfahren nach Anspruch 6,
**dadurch gekennzeichnet,**
**dass** die dielektrische Verlängerung (12) eine Dielektrizitätskonstante von ε > 2, bevorzugt von ε > 3, besonders bevorzugt von ε > 4 hat, und
**dass** die dielektrische Verlängerung (12) eine Länge von 20cm bis 120cm, bevorzugt von 30cm bis 100cm, besonders bevorzugt von 40cm bis 80cm hat.

8. Verfahren nach einem der Ansprüche 6 oder 7,
**dadurch gekennzeichnet,**
**dass** seitlich die dielektrische Verlängerung (12) verlassende Mikrowellenstrahlung von einem seitlich angeordneten Mikrowellenabsorber (13) absorbiert wird.

9. Verfahren nach Anspruch 8,
**dadurch gekennzeichnet,**
**dass** der Mikrowellenabsorber (13) aus einen stark dämpfenden, nicht reflektierenden Material besteht.

10. Verfahren nach einem der Ansprüche 6 bis 9,
**dadurch gekennzeichnet,**
**dass** das zu untersuchende Objekt (11) eine Brust einer Patientin (10) ist,
**dass** die Brust (11) an einem Ende der dielektrischen Verlängerung (12) angeordnet wird, und
**dass** sich zwischen der Brust (11) und der dielektrischen Verlängerung (12) im Wesentlichen keine Luft befindet.

## Claims

1. A measuring device with a microwave transmitter (2), a microwave receiver (3), at least one antenna (6), a control device (4) and a dielectric extension (12),
wherein the dielectric extension (12) is disposed between the antenna (6) and an object (10) to be investigated,
wherein the control device (4) controls the microwave transmitter (2) and the microwave receiver (3),
wherein the microwave transmitter (2) transmits a microwave signal by means of the antenna (6) and the dielectric extension (12) into the object (11) to be investigated,
wherein the object (11) to be investigated scatters the microwave signal,
wherein the microwave receiver (3) receives the scattered microwave signal by means of the antenna (6) and the dielectric extension (12),
wherein the length and the dielectric constant of the dielectric extension (12) are dimensioned in such a manner that the object (11) to be investigated is disposed in the remote field of the antenna (6), and
wherein the remote field of the antenna (6) is reached after a travel by the microwave signal through the dielectric extension (12) of six wavelengths.

2. The measuring device according to claim 1,
**characterised in that**
the dielectric extension (12) has a dielectric constant of ε > 2, preferably of ε > 3, by particular preference of ε > 4, and
that the dielectric extension (12) has a length of 20 cm to 120 cm, preferably from 30 cm to 100 cm, by particular preference from 40 cm to 80 cm.

3. The measuring device according to claim 1 or 2,
**characterised in that**
the dielectric extension (12) provides a laterally disposed microwave absorber (13).

4. The measuring device according to claim 3,
**characterised in that**
the microwave absorber (13) comprises a strongly attenuating, non-reflecting material.

5. The measuring device according to any one of claims 1 to 4,
**characterised in that**
the object to be investigated is a breast (11) of a female patient (10),
that the breast is disposed at one end of the dielectric extension (12), and
that substantially no air is disposed between the breast (11) and the dielectric extension (12).

6. A method for investigating an object with a microwave transmitter (2), a microwave receiver (3), at least one antenna (6), a control device (4) and a dielectric extension (12),
wherein the microwave transmitter (2) and the microwave receiver (3) are controlled by the control device (4),
wherein the following steps are implemented:
- transmission of the microwave signal by the microwave transmitter (2) by means of the antenna (6) ;
- extension of the effective path of the microwave signal by the dielectric extension (12), wherein the length and the dielectric constant of the dielectric extension (12) are dimensioned in such a manner that the object (11) to be investigated is disposed in the remote field of the antenna (6), wherein the remote field of the antenna (6) is reached after a travel by the microwave signal through the dielectric extension (12) of six wavelengths;
- scattering of the microwave signal by the object (11) to be investigated; and
- reception of the scattered microwave signal by the microwave receiver (3) by means of the antenna (6).

7. The method according to claim 6,
**characterised in that**
the dielectric extension (12) has a dielectric constant of ε > 2, preferably of ε > 3, by particular preference of ε > 4, and
that the dielectric extension (12) has a length of 20 cm to 120 cm, preferably from 30 cm to 100 cm, by particular preference from 40 cm to 80 cm.

8. The method according to any one of claims 6 or 7,
**characterised in that**
the microwave radiation leaving the dielectric extension (12) laterally is absorbed by a laterally disposed microwave absorber (13).

9. The method according to claim 8,
**characterised in that**
the microwave absorber (13) comprises a strongly attenuating, non-reflecting material.

10. The measuring device according to any one of claims 6 to 9,
**characterised in that**
the object to be investigated is a breast (11) of a female patient (10),
that the breast is disposed at one end of the dielectric extension (12), and
that substantially no air is disposed between the breast (11) and the dielectric extension (12).

## Revendications

1. Dispositif de mesure comportant un émetteur micro-ondes (2), un récepteur micro-ondes (3), au moins une antenne (6), un dispositif de commande (4) et une extension diélectrique (12),
où l'extension diélectrique (12) est disposée entre l'antenne (6) et un objet à examiner (10),
où le dispositif de commande (4) commande l'émetteur micro-ondes (2) et le récepteur micro-ondes (3),
où l'émetteur micro-ondes (2) émet un signal micro-ondes au moyen de l'antenne (6) et de l'extension diélectrique (12) vers l'objet à examiner (11),
où l'objet à examiner (11) diffuse le signal micro-ondes,
où le récepteur micro-ondes (3) reçoit le signal micro-ondes diffusé au moyen de l'antenne (6) et de l'extension diélectrique (12),
où la longueur et la constante diélectrique de l'extension diélectrique (12) sont dimensionnées de telle manière que l'objet à examiner (11) soit situé dans la zone de Fraunhofer de l'antenne (6), et
où la zone de Fraunhofer de l'antenne (6) est atteinte après parcours de six longueurs d'onde par le signal micro-ondes dans l'extension diélectrique (12).

2. Dispositif de mesure selon la revendication 1,
**caractérisé**
**en ce que** l'extension diélectrique (12) présente une constante diélectrique ε > 2, de préférence > 3, et tout particulièrement > 4, et
**en ce que** l'extension diélectrique (12) a une longueur comprise entre 20 cm et 120 cm, de préférence comprise entre 30 cm et 100 cm, et tout particulièrement comprise entre 40 cm et 80 cm.

3. Dispositif de mesure selon la revendication 1 ou la revendication 2,
**caractérisé**
**en ce que** l'extension diélectrique (12) dispose d'un absorbeur de micro-ondes (13) disposé latéralement.

4. Dispositif de mesure selon la revendication 3,
**caractérisé**
**en ce que** l'absorbeur de micro-ondes (13) est constitué d'un matériau fortement atténuateur et non réfléchissant.

5. Dispositif de mesure selon l'une des revendications 1 à 4,
**caractérisé**
**en ce que** l'objet à examiner est la poitrine (11) d'une patiente (10),
**en ce que** la poitrine est située à une extrémité de l'extension diélectrique (12),
et **en ce que** l'air fait sensiblement défaut entre la poitrine (11) et l'extension diélectrique (12).

6. Procédé d'examen d'un objet au moyen d'un émetteur micro-ondes (2), d'un récepteur micro-ondes (3), d'au moins une antenne (6), d'un dispositif de commande (4) et d'une extension diélectrique (12),
où l'émetteur micro-ondes (2) et le récepteur micro-ondes (3) sont commandés par le dispositif de commande (4),
où les étapes suivantes sont exécutées :
- émission d'un signal micro-ondes par l'émetteur micro-ondes (2) au moyen de l'antenne (6) ;
- extension du trajet effectif du signal micro-ondes dans l'extension diélectrique (12), la longueur et la constante diélectrique de l'extension diélectrique (12) étant dimensionnées de telle manière que l'objet à examiner (11) soit situé dans la zone de Fraunhofer de l'antenne (6) ;
- diffusion du signal micro-ondes par l'objet à examiner (11), et
- réception du signal micro-ondes diffusé par le récepteur micro-ondes (3) au moyen de l'antenne (6).

7. Procédé selon la revendication 6,
**caractérisé**
**en ce que** l'extension diélectrique (12) présente une constante diélectrique ε > 2, de préférence > 3, et tout particulièrement > 4, et en ce que l'extension diélectrique (12) a une longueur comprise entre 20 cm et 120 cm, de préférence comprise entre 30 cm et 100 cm, et tout particulièrement comprise entre 40 cm et 80 cm.

8. Procédé selon l'une des revendications 6 ou 7,
**caractérisé**
**en ce que** le rayonnement micro-ondes quittant latéralement l'extension diélectrique (12) est absorbé par un absorbeur de micro-ondes (13) disposé latéralement.

9. Procédé selon la revendication 8,
**caractérisé**
**en ce que** l'absorbeur de micro-ondes (13) est constitué d'un matériau fortement atténuateur et non réfléchissant.

10. Procédé selon l'une des revendications 6 à 9,
**caractérisé**
**en ce que** l'objet à examiner (11) est la poitrine d'une patiente (10),
**en ce que** la poitrine (11) est située à une extrémité de l'extension diélectrique (12),
et **en ce que** l'air fait sensiblement défaut entre la poitrine (11) et l'extension diélectrique (12).
